(19) Europäisches Patentamt
European Patent Office
Office européen des brevets

(11) **EP 4 287 840 B1**

(12) **FASCICULE DE BREVET EUROPEEN**

(45) Date de publication et mention de la délivrance du brevet:
**01.04.2026   Bulletin 2026/14**

(21) Numéro de dépôt: **22708194.0**

(22) Date de dépôt: **07.02.2022**

(51) Classification Internationale des Brevets (IPC):
**A23K 10/16** (2016.01)    **A23K 10/30** (2016.01)
**A23L 33/105** (2016.01)    **A61K 35/748** (2015.01)
**A61K 36/02** (2006.01)    **A61K 36/03** (2006.01)

(52) Classification Coopérative des Brevets (CPC):
(C-Sets disponibles)
**A23K 10/30; A23K 10/16; A23L 33/105;
A61K 35/748; A61K 36/03**    (Cont.)

(86) Numéro de dépôt international:
**PCT/FR2022/050226**

(87) Numéro de publication internationale:
**WO 2022/167772 (11.08.2022 Gazette 2022/32)**

(54) **COMPOSITION POUR LA NUTRITION OU LA BOISSON D'UN ANIMAL NON HUMAIN**

ZUSAMMENSETZUNG ZUR ERNÄHRUNG ODER ZUM TRINKEN EINES NICHTMENSCHLICHEN TIERES

COMPOSITION FOR THE NUTRITION OR DRINK OF A NON-HUMAN ANIMAL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats de validation désignés:
**MA**

(30) Priorité:  **08.02.2021  FR 2101179**

(43) Date de publication de la demande:
**13.12.2023   Bulletin 2023/50**

(73) Titulaire: **Agro Innovation International
35400 Saint-Malo (FR)**

(72) Inventeurs:
• **LAZA KNOERR, Anca L.
35400 SAINT-MALO (FR)**
• **POINT, Sandra
35540 PLERGUER (FR)**
• **DE TONNAC, Auriane
35170 BRUZ (FR)**
• **DUMARGUE, Philippe
22440 PLOUFRAGAN (FR)**

(74) Mandataire: **Cabinet Beau de Loménie
103, rue de Grenelle
75340 Paris Cedex 07 (FR)**

(56) Documents cités:
**DE-A1- 19 608 563        IT-A1- PD20 110 179
KR-A- 20200 129 841**

• **MAKKAR HARINDER P S ET AL: "Seaweeds for livestock diets: A review", ANIMAL FEED SCIENCE AND TECHNOLOGY, ELSEVIER, AMSTERDAM, NL, vol. 212, 25 September 2015 (2015-09-25), pages 1 - 17, XP029389831, ISSN: 0377-8401, DOI: 10.1016/ J.ANIFEEDSCI.2015.09.018**
• **PRAVEEN M AJANTH ET AL: "An overview of extraction and purification techniques of seaweed dietary fibers for immunomodulation on gut microbiota", TRENDS IN FOOD SCIENCE AND TECHNOLOGY, ELSEVIER SCIENCE PUBLISHERS, GB, vol. 92, 14 August 2019 (2019-08-14), pages 46 - 64, XP085817784, ISSN: 0924-2244, [retrieved on 20190814], DOI: 10.1016/ J.TIFS.2019.08.011**

- **BELAY AMHA ET AL: "Spirulina (Arthrospira): Potential application as an animal feed supplement", JOURNAL OF APPLIED PHYCOLOGY, KLUWER, DORDRECHT, NL, vol. 8, no. 4-5, 1 January 1996 (1996-01-01), pages 303 - 311, XP009125312, ISSN: 0921-8971, DOI: 10.1007/BF02178573**

(52) Classification Coopérative des Brevets (CPC): (Cont.)

C-Sets
**A61K 36/03, A61K 2300/00**

**Description**

Domaine Technique

La présente invention concerne le domaine de l'alimentation et/ou boisson animale non humaine, et en particulier de l'association des extraits des algues spiruline et *Ascophyllum nodosum* en vue de l'alimentation et/ou boisson animale non humaine d'animaux d'élevage, plus particulièrement de rente.

Technique antérieure

La demande WO 2004/080196 décrit l'utilisation de produits dérivés de macroalgues, de microalgues, de plantes et/ou de champignons dans l'alimentation animale, en remplacement de produits dérivés des animaux en particulier de poissons, en raison de leur forte teneur en AGPI-LC (acides gras poly-insaturés à longue chaine) tels que le DHA (acide docosahexaenoique) et l'EPA (acide eicosapentaenoique). Parmi les microalgues est citée la spiruline et parmi les macroalgues, l'Ascophyllum. Toutefois ces algues sont citées parmi une longue liste d'autres microalgues ou macroalgues possibles. En outre aucun exemple spécifique ne comprend le mélange de ces deux algues. Par ailleurs ces algues ne sont pas utilisés brutes ou sous forme d'extrait mais sous forme de produits dérivés.

La demande WO 2001/22822 décrit l'utilisation d'une composition comprenant une cytokine issue d'*Ascophyllum nodosum* en mélange avec des nutriments à base de minéraux, un mélange de vitamine B et de l'acide acétyle salicylique ou un de ses sels, sous la forme d'une boisson ou d'une solution injectable, pour améliorer l'immunité des animaux. La cytokine issue d'*Ascophyllum nodosum* est obtenue par extraction qui en utilise un procédé très particulier. La demande décrit la possible présence dans la composition d'autres ingrédients tels que la spiruline, qui est citée dans une longue liste d'ingrédients, sans qu'aucun exemple d'association de ces deux algues (*Ascophyllum nodosum* et spiruline) ne soit divulgué.

La demande de brevet EP1570845 décrit l'utilisation de polysaccharides acides en tant qu'agent pour améliorer la production de protéine morphogénétique osseuse ou améliorer l'ostéogénèse. Le polysaccharide acide peut être dérivé d'algues telles qu'entre autres la spiruline ou *l'Ascophyllum nodosum.* Cependant, ce document ne divulgue aucun exemple spécifique contenant des polysaccharides acides, en particulier sulfatés provenant à la fois d'*Ascophyllum nodosum* et de spiruline.

La demande KR2020-0129841 décrit dans ses exemples ([0051] - [0059]) un modèle animal (rat) auquel on administre par voie orale un extrait aqueux de spiruline (SP à 800mg/kg/jour) ou *d'Ascophilum Nodosum* (AN à 100mg/kg/jour ou 200mg/kg/jour) mélangé avec de l'eau. Toutefois les extraits aqueux de spiruline et *d'Ascophilum nodosum* sont utilisés séparément. En outre leur utilisation n'est pas pour l'amélioration du bien-être chez un animal mais pour diminuer ou supprimer la toxicité du plomb ingéré par les animaux, le but de ces tests étant l'obtention d'un médicament pour l'être humain.

En outre bien que cette demande décrive bien des extraits aqueux de spiruline ou *d'Ascophilum Nodosum* ([0032]) utilisés dans la boisson d'animaux, elle ne décrit pas d'extrait protéique de spiruline (et encore moins un hydrolysat protéique) ni d'extrait polysaccharidique d'Ascophilum nodosum.

Enfin les rats ne sont pas des animaux d'élevage et encore moins des animaux de rente.

La demande de brevet DE 19608563 décrit des compositions alimentaires comprenant l'association de spiruline brute et *d'Ascophilum nodosum* brut et de lithothamnium.

Toutefois il n'est nulle part indiqué dans cette demande que ces compositions sont destinées à l'alimentation des animaux en particulier des animaux d'élevage et encore moins des animaux de rente.

Ces compositions sont en fait destinées aux humains chez qui elles ont été testées (exemples).

La demande ITPD20-110179 décrit une composition pour contrôler la dysglycémie chez un animal.

Toutefois il ne s'agit pas d'un animal d'élevage et encore moins de rente (tels que volaille, ruminant, porcin et/ou animaux aquatiques) mais d'un animal domestique du type chat ou chien.

En effet les animaux d'élevage ne souffrent pas de dysglycémie. C'est une maladie typique des animaux de compagnie. La composition décrite dans cette demande n'est donc pas adaptée aux animaux d'élevage ni aux animaux de rente.

En outre la composition décrite dans cette demande doit contenir un extrait de *Gymnema Sylvestre,* un extrait de *Momordica Charantia* et du zinc, ce qui n'est pas le cas de la composition selon l'invention.

En outre cette demande ne décrit pas d'extrait de spiruline ou *d'Ascophilum Nodosum* encore moins un extrait protéique de spiruline (et encore moins un hydrolysat protéique) ou un extrait polysaccharidique *d'Ascophilum nodosum.*

Or, les inventeurs se sont aperçus de façon surprenante que l'association d'extrait polysaccharidique *d'Ascophyllum nodosum* et d'extrait protéique de spiruline avait une action synergique pour améliorer les performances zootechniques des animaux non humains d'élevage, plus particulièrement de rente, avantageusement d'un ruminant et/ou d'un monogastrique telle qu'une volaille ou un porc et/ou d'un animal aquatique tel qu'un poisson ou un crustacé, lorsque l'association était administrée dans une composition alimentaire ou une boisson.

Exposé de l'invention

La présente invention concerne donc une composition pour la nutrition ou la boisson d'un animal non humain d'élevage, plus particulièrement de rente, avantageusement d'un ruminant et/ou d'un monogastrique telle qu'une volaille ou un porc et/ou d'un animal aquatique tel qu'un poisson ou un crustacé, comprenant l'association d'un hydrolysat protéique de spiruline et d'un extrait polysaccharidique *d'Ascophyllum nodosum.*

La composition selon l'invention comprend donc de la spiruline.

La spiruline est une microalgue (35$\mu$m de large et 200$\mu$m de long en moyenne) reconnaissable à sa forme particulière en spirale. Son nom scientifique est *Arthrospira.* C'est une cyanobactérie appartenant à la classe des *Cyanophyceae* (algues bleu-vert). Sa couleur est due à la présence de phycocyanine (protéine), un pigment bleu utilisé notamment en agroalimentaire. Il existe près de 1 500 espèces d'algues bleues et 36 espèces de spiruline sont comestibles. La principale espèce actuellement offerte sur le marché est la *Spirulina platensis.* La spiruline selon l'invention est donc avantageusement la *Spirulina platensis.* La spiruline contient des protéines (dont la phycocyanine), des caroténoïdes variés (bêta-carotène principalement, mais aussi cryptoxanthine, lutéine, zéaxanthine, etc.), des vitamines et des minéraux dont du fer, magnésium, calcium, vitamines A, B, E. La spiruline comprend en outre une bonne teneur en acides aminés essentiels (tels que acide aspartique, thréonine, sérine, acide glutamique, proline, glycine, alanine, cystine, valine, méthionine, isoleucine, leucine, tyrosine, phénylalanine, histidine, lysine et arginine) et de l'acide gamma-linolénique, un acide gras insaturé de la famille des oméga-6. En particulier, sa teneur en protéine est comprise entre 55 et 70% (bornes comprises), avantageusement supérieure ou égale à 60%, particulièrement entre 60 et 70% (bornes comprises), plus avantageusement entre 65 et 69% (bornes comprises), en poids par rapport au poids total de la spiruline.

De plus, la spiruline est riche en phycocyanine, le seul pigment bleu naturel pouvant servir de colorant alimentaire et auquel on attribue une activité antioxydante importante, en particulier sa teneur est supérieure ou égale à 7%, plus avantageusement supérieure ou égale à 8%. Elle contient également de la chlorophylle.

La spiruline selon l'invention peut donc être utilisée sous forme de poudre. De façon avantageuse elle n'est pas utilisée sous forme d'hydrogel. De façon encore plus avantageuse, elle ne contient pas de mucilage. Dans un mode de réalisation particulièrement avantageux, la spiruline selon l'invention n'est pas utilisée en association avec ou dans une composition contenant un extrait de *Gymnema Sylvestre* et/ou un extrait de *Momordica Charantia* et/ou du zinc.

La spiruline se trouve sous la forme d'un hydrolysat protéique.

En particulier l'extraction est mise en œuvre par macération avantageusement dans un solvant aqueux, plus avantageusement de l'eau, plus particulièrement en une teneur de 100g de spiruline déshydratée dans 900g de solvant, en particulier à une température comprise entre 30 et 80°C (bornes comprises), plus avantageusement entre 50 et 70°C (bornes comprises), en particulier de 60°C, pendant une durée avantageusement comprise entre 1 heure et 6 heures (bornes comprises), plus avantageusement entre 2 heures et 4 heures (bornes comprises). Avantageusement l'hydrolysat protéique est obtenu par hydrolyse de l'extrait protéique, en particulier par hydrolyse enzymatique, plus avantageusement à l'aide d'une enzyme endoprotéase telle que les endoprotéases à sérine comme par exemple la subtilisine, avantageusement commercialisée par Novozymes sous la dénomination commerciale Novo-Pro® D. L'hydrolyse a lieu avantageusement en solution aqueuse, en particulier à un pH compris entre 7 et 11 (bornes comprises), plus particulièrement entre 7 et 10 (bornes comprises), encore plus particulièrement entre 8 et 9,5 (bornes comprises), en particulier de 8, avantageusement à une température comprise entre 55 et 75°C (bornes comprises), plus avantageusement entre 55 et 65°C (bornes comprises), en particulier de 60°C.

Dans un mode de réalisation avantageux, l'extrait se trouve sous forme liquide.

L'hydrolysat protéique contient avantageusement des polypeptides, des peptides et des acides aminés (tels que acide aspartique, thréonine, sérine, acide glutamique, proline, glycine, alanine, cystine, valine, méthionine, isoleucine, leucine, tyrosine, phénylalanine, histidine, lysine et arginine), avantageusement le poids moléculaire des polypeptides et des peptides peut aller de moins de 150 Da, à plus de 20 000 Da. De façon avantageuse, l'hydrolysat protéique comprend plus de 85% en poids de polypeptides, peptides et acide aminés ayant un poids moléculaire inférieur ou égal à 5000 Da, avantageusement plus de 90% en poids, en particulier plus de 92% en poids par rapport au poids total de polypeptides, peptides et acides aminés de l'hydrolysat protéique. De façon avantageuse, l'hydrolysat protéique comprend plus de 70% en poids de polypeptides, peptides et acides aminés ayant un poids moléculaire inférieur ou égal à 1000 Da, avantageusement plus de 75% en poids, en particulier plus de 80% en poids, par rapport au poids total de polypeptides, peptides et acides aminés de l'hydrolysat protéique. De façon avantageuse, l'hydrolysat protéique comprend plus de 50% en poids de polypeptides, peptides et acides aminés ayant un poids moléculaire inférieur ou égal à 500 Da, avantageusement plus de 60% en poids, en particulier plus de 65% en poids, par rapport au poids total de polypeptides, peptides et acides aminés de l'hydrolysat protéique.

Avantageusement la composition selon l'invention comprend entre 0,01 et 0,5 % (bornes incluses) en poids par rapport au poids total de la composition, avantageusement entre 0,03 et 0,4,% (bornes incluses) en poids par rapport au poids total de la composition, encore plus avantageusement entre 0,02 et 0,2% (bornes incluses) en poids par rapport au poids total de la composition, d'hydrolysat protéique de spiruline. La teneur en spiruline de la composition dépend en particulier de l'animal auquel l'aliment ou la boisson est destiné.

Ainsi, en particulier la composition selon l'invention comprend 0,2% en poids par rapport au poids total de la composition, d'hydrolysat protéique de spiruline, lorsque la composition est destinée aux porcins, plus avantageusement lorsqu'elle est destinée aux porcs ou aux porcelets, sevrés ou non, en particulier lorsqu'elle est destinée aux porcelets, sevrés ou non. Plus particulièrement la composition selon l'invention comprend entre 0,02% et 0,1% (bornes incluses) en poids par rapport au poids total de la composition, d'hydrolysat protéique de spiruline, lorsque la composition est destinée aux oiseaux, en particulier d'élevage, plus particulièrement aux volailles, plus avantageusement lorsqu'elle est destinée aux gallinacés tels que les poulets ou les poussins.

De façon avantageuse, la teneur en hydrolysat protéique de spiruline, administrée à des ruminants, en particulier à des vaches laitières, à l'aide de la composition selon l'invention, est comprise entre 2 et 70g/J (bornes incluses), en particulier entre 5 et 50g/J (bornes incluses), plus avantageusement elle est de 5g/J, pour une ration d'environ 15kg de fourrage par jour.

La composition selon l'invention comprend en outre de l'*Ascophyllum nodosum*.

L'*Ascophyllum nodosum,* aussi dite Goémon noir, algue noueuse, est une macroalgue (elle peut atteindre plus de 1,50 m de longueur) de couleur brune qui appartient à la classe des *Phaeophyceae*. C'est la seule représentante, actuellement reconnue, du genre *Ascophyllum,* dans la famille des *Fucaceae.*

Elle contient de hautes teneurs en macro-éléments, (tels que les éléments N, P, K, Ca, Mg, S) et oligo-éléments (par exemple Mn, Cu, Fe, Zn, etc.). Elle contient aussi des phytohormones telles que bétaïne, cytokinine, auxine et gibbérelline (stimulateurs de croissance), du mannitol, des acides organiques, des polysaccharides, des acides aminés et des protéines.

L'*Ascophyllum nodosum* selon l'invention peut donc être utilisée sous forme de poudre.

De façon avantageuse l'*Ascophyllum nodosum* selon l'invention n'est pas utilisé sous forme d'hydrogel. De façon encore plus avantageuse, il ne contient pas d'AOS (alginatooligosaccharides). Dans un mode de réalisation particulièrement avantageux, l'*Ascophyllum nodosum* selon l'invention n'est pas utilisé en association avec ou dans une composition contenant un extrait de *Gymnema Sylvestre* et/ou un extrait de *Momordica Charantia* et/ou du zinc.

Toutefois, l'*Ascophyllum nodosum* se trouve sous la forme d'un extrait polysaccharidique (en particulier hydrosoluble), avantageusement l'extrait ne contient pas de cytokine, plus avantageusement l'extrait a été clarifié, encore plus avantageusement déshydraté.

En particulier l'extraction est mise en œuvre par macération avantageusement dans un solvant aqueux, plus avantageusement de l'eau, en particulier en une teneur de 200g d'*Ascophyllum nodosum* déshydratée dans 800g de solvant, en particulier à une température comprise entre 30 et 95°C (bornes comprises), plus avantageusement entre 45 et 90°C (bornes comprises), pendant une durée avantageusement comprise entre 1 heure et 6 heures (bornes comprises), plus avantageusement entre 2 heures et 4 heures (bornes comprises).

Dans un mode de réalisation avantageux, l'extrait se trouve sous forme liquide. Dans un mode de réalisation encore plus avantageux, il se trouve sous forme de poudre.

Avantageusement la composition selon l'invention comprend entre 0,05 et 0,5 % (bornes incluses) en poids par rapport au poids total de la composition, avantageusement entre 0,06 et 0,15% (bornes incluses) en poids par rapport au poids total de la composition, plus avantageusement entre 0,01 et 0,1% (bornes incluses) en poids par rapport au poids total de la composition, d'extrait polysaccharidique d'*Ascophyllum nodosum.* La teneur en *Ascophyllum nodosum* de la composition dépend en particulier de l'animal auquel l'aliment ou la boisson est destiné.

En particulier la composition selon l'invention comprend entre 0,02% et 0,1% (bornes incluses) en poids par rapport au poids total de la composition, d'extrait polysaccharidique d'*Ascophyllum nodosum,* lorsque la composition est destinée aux porcins, plus avantageusement lorsqu'elle est destinée aux porcs ou aux porcelets, sevrés ou non en particulier lorsqu'elle est destinée aux porcelets, sevrés ou non.

Plus particulièrement la composition selon l'invention comprend entre 0,01% et 0,05% (bornes incluses) en poids par rapport au poids total de la composition, d'extrait polysaccharidique d'*Ascophyllum nodosum,* lorsque la composition est destinée aux oiseaux, en particulier d'élevage, plus particulièrement aux volailles, plus avantageusement lorsqu'elle est destinée aux gallinacés tels que les poulets ou les poussins.

De façon avantageuse, la teneur en extrait polysaccharidique d'*Ascophyllum nodosum,* administrée à des ruminants, en particulier à des vaches laitières, à l'aide de la composition selon l'invention, est comprise entre 5 et 50g/J (bornes incluses), en particulier entre 10 et 20g/J (bornes incluses), plus avantageusement elle est de 10g/J, pour une ration d'environ 15kg de fourrage par jour.

Avantageusement le ratio en poids hydrolysat protéique de spiruline / extrait polysaccharidique d'*Ascophyllum nodosum* est compris dans la gamme 0,02-100 (bornes incluses), avantageusement 0,2 - 15 (bornes incluses), en particulier 0,5 - 20 (bornes incluses) plus particulièrement entre ½ - 3/1 (bornes incluses). Ce ratio dépend en particulier de l'animal auquel l'aliment ou la boisson est destiné.

En particulier ce ratio est compris dans la gamme 2-10 (bornes incluses), lorsque la composition est destinée aux porcins, plus avantageusement lorsqu'elle est destinée aux porcs ou aux porcelets, sevrés ou non, en particulier lorsqu'elle est destinée aux porcelets, sevrés ou non.

Plus particulièrement ce ratio est compris dans la gamme 2-20 (bornes incluses), lorsque la composition est destinée aux oiseaux, en particulier d'élevage, plus particulièrement aux volailles, plus avantageusement lorsqu'elle est destinée aux gallinacés tels que les poulets ou les poussins. De façon avantageuse, ce ratio est compris dans la gamme 0,04-14 (bornes incluses), avantageusement 0,25-5 (bornes incluses), plus avantageusement 0,5-2,5 (bornes incluses), encore plus avantageusement il est de 0,5, lorsque la composition est destinée aux ruminants, en particulier aux vaches laitières, plus particulièrement ayant une ration journalière de 15kg de fourrage.

La composition selon l'invention est donc destinée à une administration par voie orale aux animaux. Il peut s'agir d'une composition alimentaire ou d'une boisson, avantageusement d'une composition alimentaire.

La composition selon l'invention peut en outre comprendre du lithothamne. Elle peut par ailleurs comprendre d'autres ingrédients connus de l'homme du métier pour la nutrition ou la boisson des animaux, tels que par exemple du fourrage dans le cas des ruminants ou de l'eau dans le cas des boissons et/ou des rafles dans le cas des volailles.

Dans un mode de réalisation avantageux, la composition selon l'invention ne contient pas d'extrait de *Gymnema Sylvestre* et/ou d'extrait de *Momordica Charantia* et/ou de zinc.

La composition selon l'invention peut avoir toute forme appropriée pour l'alimentation ou la boisson des animaux non humains, en particulier une forme solide, une forme de poudre ou de granulé ou une forme liquide ou de gel.

Elle peut ainsi être ajoutée directement dans la ration des animaux non humains, ou être ajoutée à des prémix ou compléments alimentaires ou à des blocs ou seaux nutritionnels.

Dans un mode de réalisation avantageux, elle est ajoutée à la ration journalière d'un animal non humain, en particulier d'un ruminant tel que par exemple une vache laitière, avantageusement à raison de 5mg/jour de composition pour 1 kg de matière sèche de ration.

Cette ration peut être par exemple pour les ruminants composée de fourrages de tous types et sous toutes leurs formes (verts, déshydratés, ensilés, agglomérés, etc.) comme l'herbe et les autres graminées fourragères, les céréales fourragères (orge, maïs, avoine, blé, sorgho, soja, seigle), les légumineuses (pois, féverole, lupin, soja, luzerne, sainfoin, trèfles), les racines, tubercules et leurs sous-produits (betteraves, pulpe de betteraves, pomme de terre, pulpe de pomme de terre, etc.), le chou, le colza, le tournesol, les déchets de végétaux (fanes, rafles, balles de céréales, son, épis de maïs égrenés, bagasse) et les fécules, les sous-produits d'industrie agro-alimentaire (amidonnerie, féculerie, éthanolerie, brasserie, meunerie, etc.), ainsi que des tourteaux de graines oléagineuses, des sirops, et des matières alimentaires azotées telles que l'urée et ses dérivés (biuret, uréides) et les sels ammoniacaux.

De préférence, la ration appropriée pour les ruminants selon l'invention comprend des fourrages, de préférence de l'ensilage de maïs représentant typiquement de 10 à 50% de la matière sèche ingérée, de préférence quotidiennement, associé de préférence à d'autres fourrages tels que du foin, de la paille ou de l'ensilage d'herbe ou de céréales et complémenté de préférence par des aliments concentrés tels que des céréales, des tourteaux d'oléagineux ou des aliments composés.

En particulier, il s'agit d'un mélange d'ensilage de maïs, de foin et de concentrés (constitué par exemple de blé, maïs, d'orge, de pulpe de betterave, de son de blé et de mélasse), par exemple en un ratio 50 :30 :20, ou de de foin, de concentré et d'amidon de maïs, par exemple à un ratio 77 :9 :12 :2.

La composition est destinée à un animal non humain d'élevage, plus particulièrement de rente, avantageusement un ruminant et/ou un monogastrique et/ou un animal aquatique. En particulier, il peut s'agir d'un mammifère.

Par « animal de rente » ou « animal de production », on entend au sens de la présente invention tout animal élevé ou gardé pour sa rentabilité, c'est-à-dire « la production de denrées alimentaires, de laine, de peaux ou d'autres fins agricoles ».

Parmi les animaux, les herbivores et en particulier les ruminants sont des mammifères qui ont une alimentation à base de fibres végétales contenant parmi les fibres solubles des fibres insolubles de type celluloses et hémicelluloses, fibres très peu digestibles.

Le ruminant a la particularité de digérer par l'intermédiaire d'une « cuve de fermentation », le rumen (130-180 litres) interposé dans la partie antérieure du tube digestif. Le rumen contient une grande variété de microflore de bactéries et de protozoaires qui effectuent une prédigestion fermentaire prioritaire et très efficace. La flore microbienne conditionne donc la digestibilité des glucides, des protéines, l'auto- approvisionnement en vitamines ou autres nutriments.

Dans un mode de réalisation avantageux, la composition selon la présente invention est destinée à la nutrition et/ou à la boisson des mammifères non humains d'élevage, plus particulièrement de rente, en particulier des herbivores (tels que ruminants, chevaux, lagomorphes) et plus particulièrement des ruminants.

Les ruminants incluent par exemple les bovins, les ovins, les caprins, les cervidés et les camélidés.

Par "bovin" on entend au sens de la présente invention une sous-famille des bovidés comprenant plusieurs espèces importantes d'animaux d'élevage. Les bovins incluent en particulier la vache, en particulier la vache laitière, la vache allaitante, la génisse, le veau, le broutard, le taurillon, le bœuf, le bœuf à l'engrais, le taureau, le buffle, le yack, le gayal et le banteng.

Par "ovin", on entend au sens de la présente invention les herbivores ruminants du genre Ovis. Les ovins incluent en particulier le mouflon, le mouton, la brebis, la vacive et l'agneau.

Par "caprin", on entend au sens de la présente invention les herbivores ruminants du genre Capra. Les caprins incluent en particulier la chèvre, le bouc, le chevreau et le bouquetin.

Par "cervidé", on entend au sens de la présente invention les ruminants de la famille des Cervidae, portant des bois. Les cervidés incluent en particulier, le cerf, le hère, le daguet, la biche, le faon, le chevreuil, le brocard, la chevrette, le renne, le daim, la daine et l'élan.

Par "camélidé", on entend au sens de la présente invention des mammifères artiodactyles de la famille des Camelidae. Les camélidés incluent en particulier le dromadaire, le chameau, la chamelle, le lama et l'alpaga. Dans un mode de réalisation avantageux, le ruminant selon l'invention est un bovin, en particulier, il est choisi parmi la vache, en particulier la vache laitière, le veau, le broutard, le veau sous la mère, le bœuf et le bœuf à l'engrais et plus particulièrement de préférence la vache laitière.

Dans un mode de réalisation avantageux, la composition selon la présente invention est destinée à la nutrition et/ou à la boisson d'un animal aquatique.

Les animaux aquatiques peuvent comprendre les poissons, en particulier d'eau douce, d'eau saumâtre ou d'eau salée, d'élevage, en particulier de rente, tels que les saumons, les truites, les bars, les carpes, les tilapias, les carassins, les catlas, les rohus, les dorades, les maigres, les turbots, les esturgeons et les pangas.

Les animaux aquatiques peuvent aussi comprendre les crustacés tels que les crevettes ou les écrevisses.

Dans un mode de réalisation avantageux, la composition selon la présente invention est destinée à la nutrition et/ou à la boisson des mammifères non humains d'élevage, plus particulièrement de rente, en particulier des monogastriques, et plus particulièrement des omnivores (porcins).

Par "porcin", on entend au sens de la présente invention des mammifères de la famille des Suidae du genre *Sus*. Les porcins incluent en particulier le porc charcutier (ou porc à l'engrais), le porcelet sevré ou non, la truie, le verrat et la cochette.

Dans un mode de réalisation avantageux, la composition selon la présente invention est destinée à la nutrition et/ou à la boisson des oiseaux d'élevage, et encore plus particulièrement de rente et en particulier des volailles.

Par « volaille », on entend au sens de la présente invention des oiseaux domestiques élevés par un mammifère humain. Les volailles peuvent appartenir à l'ordre des gallinacés ou à celui des palmipèdes.

Par « gallinacé » ou « palmipède », on entend au sens de la présente invention des animaux dont les produits de chair ou de ponte sont destinés à être consommés par un mammifère humain. Les gallinacés comprennent en particulier la poule pondeuse, la caille, le coq, le dindon, la pintade, le pigeon, le faisan et plus particulièrement le poulet de chair (dont le poussin). Les palmipèdes comprennent en particulier le canard et l'oie.

La présente invention concerne en outre l'utilisation de la composition selon l'invention pour améliorer la performance zootechnique d'un ruminant et/ou d'un omnivore par exemple un porc.

Elle concerne de plus l'utilisation de la composition selon l'invention pour diminuer l'émission de gaz à effet de serre, en particulier de méthane, chez un ruminant.

Elle concerne enfin une composition selon l'invention, pour son utilisation pour améliorer le système immunitaire chez des animaux non humains d'élevage avantageusement des ruminants et/ou des monogastriques tels que les oiseaux par exemple les volailles ou les omnivores par exemple les porcs et/ou les animaux aquatiques tels que les poissons ou les crustacés, en particulier chez les poulets, les poussins, les porcs ou les porcelets.

La présente invention sera mieux comprise à la lumière de la description des figures et des exemples qui suivent qui sont donnés à titre indicatif.

Brève description des dessins

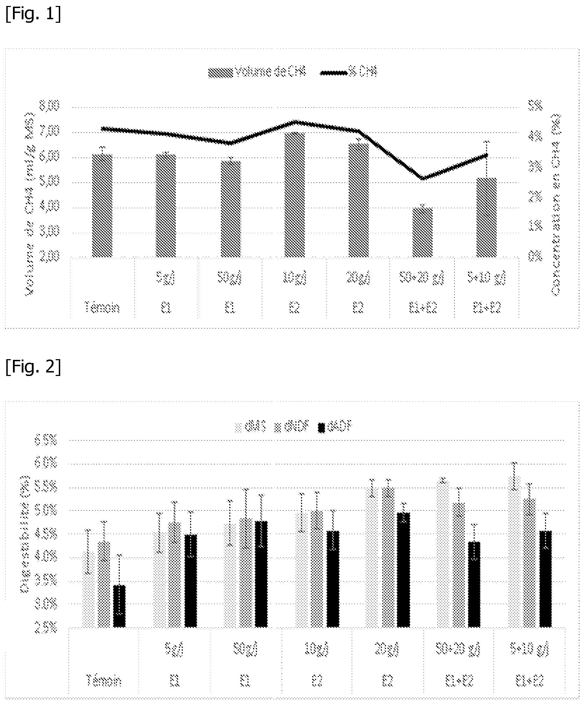

La figure 1 représente la concentration en % et le volume en ml/g de matière sèche (MS) de $CH_4$ après 24h00 de fermentation *in vitro* pour une ration hiver pour le témoin, l'hydrolysat protéique de *Spirulina platensis(E1)* à 5g/j ou 50g/j, l'extrait polysaccharidique *d'Ascophyllum nodosum* (E2) à 10g/j ou 20g/j ou l'association d'hydrolysat protéique de *Spirulina platensis* et d'extrait polysaccharidique *d'Ascophyllum nodosum* (E1+E2 à 50g/j + 20g/j ou 5g/j + 10g/j) dans les conditions de l'exemple 2.

La figure 2 représente la digestibilité de la matière sèche (dMS), de l'ADF (dADF) et du NDF (dNDF) en % après 24h de fermentation *in vitro* pour une ration hiver pour le témoin - et la composition selon l'invention, l'hydrolysat protéique de *Spirulina platensis* (E1) à 5g/j ou 50g/j, l'extrait polysaccharidique *d'Ascophyllum nodosum* (E2) à 10g/j ou 20g/j ou l'association d'hydrolysat protéique de *Spirulina platensis* et d'extrait polysaccharidique *d'Ascophyllum nodosum* (E1+E2 à 50g/j + 20g/j ou 5g/j + 10g/j) dans les conditions de l'exemple 2.

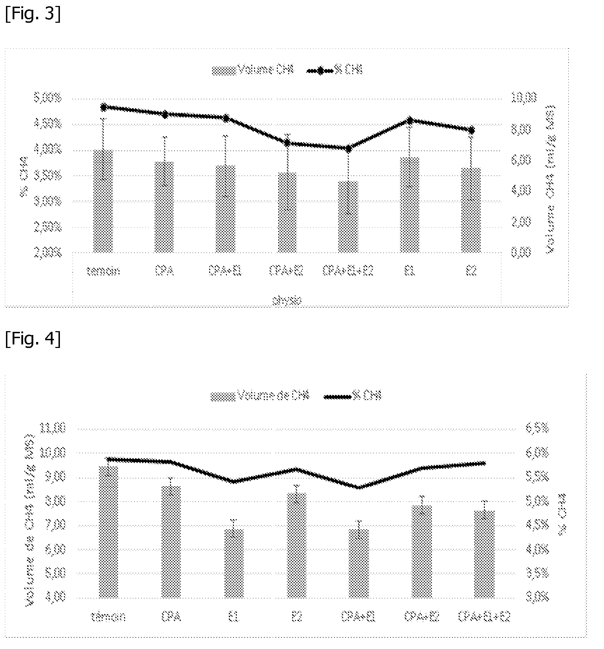

La figure 3 représente la concentration en % et le volume en ml/g de matière sèche (MS) de $CH_4$ après 24h00 de fermentation *in vitro* pour une ration hiver avec un acidogène pour le témoin, le lithothamne (CalseaPowder Advance CPA) à 80g/j, l'hydrolysat protéique de *Spirulina platensis* (E1) à 5g/j, l'extrait polysaccharidique *d'Ascophyllum nodosum* (E2) à 10g/j, l'association (simple mélange) de lithothamne et d'hydrolysat protéique de *Spirulina platensis* (CPA + E1 à 80g/J + 5g/j), l'association (simple mélange) de lithothamne et d'extrait polysaccharidique d'*Ascophyllum nodosum* (CPA + E2 à 80g/J + 10g/j) ou l'association (simple mélange) de lithothamne, d'hydrolysat protéique de *Spirulina platensis et* d'extrait polysaccharidique *d'Ascophyllum nodosum* (CPA+E1+E2 à 80g/j + 5g/j + 10g/j) dans les conditions de l'exemple 2.

La figure 4 représente la concentration en % et le volume en ml/g de matière sèche (MS) de $CH_4$ après 24h00 de fermentation *in vitro* pour une ration hiver avec un acidogène pour le témoin, le lithothamne (CalseaPowder Advance CPA) à 80g/j, l'hydrolysat protéique de *Spirulina platensis* (E1) à 5g/j, l'extrait polysaccharidique *d'Ascophyllum nodosum* (E2) à 10g/j, l'association (imprégnation de l'extrait sur le lithothamne) de lithothamne et d'hydrolysat protéique de *Spirulina platensis* (CPA + E1 à 80g/J + 5g/j), l'association (imprégnation de l'extrait sur le lithothamne) de lithothamne et d'extrait polysaccharidique *d'Ascophyllum nodosum* (CPA + E2 à 80g/J + 10g/j) ou l'association (imprégnation des extraits sur le lithothamne) de lithothamne, d'hydrolysat protéique de *Spirulina platensis et* d'extrait polysaccharidique d'*Ascophyllum nodosum* (CPA+E1+E2 à 80g/j + 5g/j + 10g/j) dans les conditions de l'exemple 2.

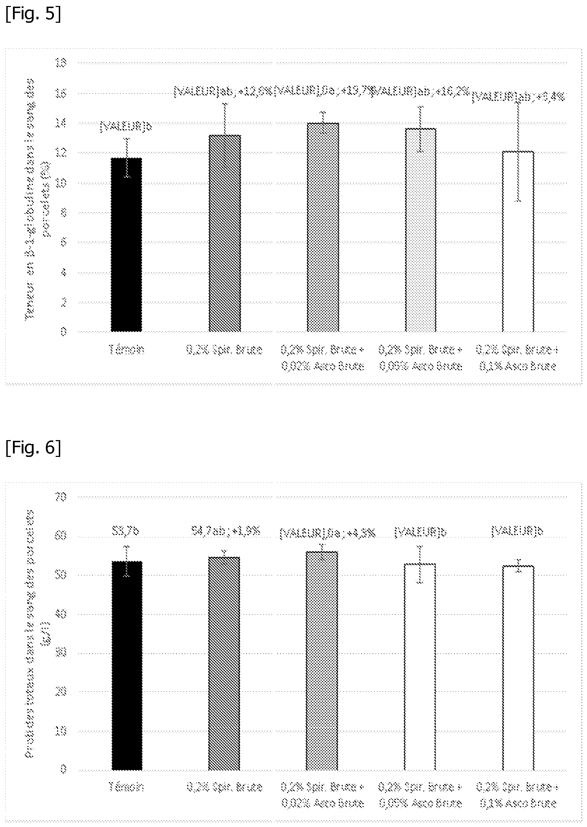

La figure 5 représente la teneur en Beta-- globuline dans le sang (%) des porcelets à J70 ayant reçu l'aliment témoin (Témoin T1), la *Spirulina platensis* brute à 0,2% (0,2% Spir. Brute T2), l'association *Spirulina platensis* brute et *d'Ascophyllum nodosum* brute à respectivement 0,2% et 0,02% (0,2% Spir. Brute + 0,02 % Asco Brute T3), à respectivement 0,2% et 0,05% (0,2% Spir. Brute + 0,05 % Asco Brute T4) ou à respectivement 0,2% et 0,1% (0,2% Spir. Brute + 0,1 % Asco Brute T5) dans les conditions de l'exemple 3 (ne faisant pas partie de l'invention). Les lettres a, b ou c associées aux chiffres indiquent les différences significatives entre traitements à 5% de confiance. Les valeurs en pourcentage indiquent l'écart d'amélioration par rapport au témoin.

La figure 6 représente la quantité de protéines totales sanguines en g/l de sang des porcelets à J70 ayant reçu l'aliment témoin (Témoin T1), la *Spirulina platensis* brute à 0,2% (0,2% Spir. Brute T2), l'association *Spirulina platensis* brute et *d'Ascophyllum nodosum* brute à respectivement 0,2% et 0,02% (0,2% Spir. Brute + 0,02 % Asco Brute T3), à respectivement 0,2% et 0,05% (0,2% Spir. Brute + 0,05 % Asco Brute T4) ou à respectivement 0,2% et 0,1% (0,2% Spir. Brute + 0,1 % Asco Brute T5) dans les conditions de l'exemple 3 (ne faisant pas partie de l'invention). Les lettres a, b ou c associées aux chiffres indiquent les différences significatives entre traitements à 5% de confiance. Les valeurs en pourcentage indiquent l'écart d'amélioration par rapport au témoin.

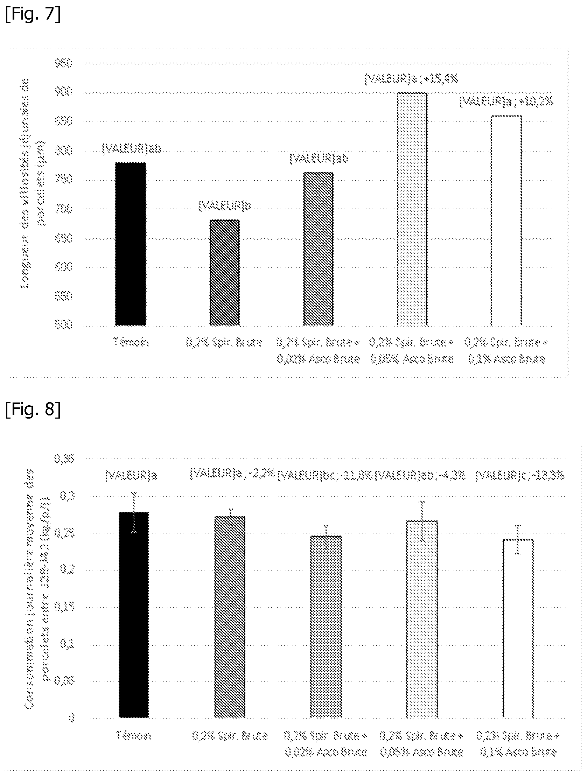

La figure 7 représente la longueur des villosités jéjunales en micromètre des porcelets à J70 ayant reçu l'aliment témoin (Témoin T1), la *Spirulina platensis* brute à 0,2% (0,2% Spir. Brute T2), l'association *Spirulina platensis* brute et *d'Ascophyllum nodosum* brute à respectivement 0,2% et 0,02% (0,2% Spir. Brute + 0,02 % Asco Brute T3), à respectivement 0,2% et 0,05% (0,2% Spir. Brute + 0,05 % Asco Brute T4) ou à respectivement 0,2% et 0,1% (0,2% Spir. Brute + 0,1 % Asco Brute T5) dans les conditions de l'exemple 3 (ne faisant pas partie de l'invention). Les lettres a, b ou c associées aux chiffres indiquent les différences significatives entre traitements à 5% de confiance. Les valeurs en pourcentage indiquent l'écart d'amélioration par rapport au témoin.

La figure 8 représente la consommation journalière moyenne sur la phase de 1er âge entre J28 et J42 en kg/porcelet/j des animaux ayant reçu l'aliment témoin (Témoin T1), la *Spirulina platensis* brute à 0,2% (0,2% Spir. Brute T2), l'association *Spirulina platensis* brute et *d'Ascophyllum nodosum* brute à respectivement 0,2% et 0,02% (0,2% Spir. Brute + 0,02 % Asco Brute T3), à respectivement 0,2% et 0,05% (0,2% Spir. Brute + 0,05 % Asco Brute T4) ou à respectivement 0,2% et 0,1% (0,2% Spir. Brute + 0,1 % Asco Brute T5) dans les conditions de l'exemple 3 (ne faisant pas partie de l'invention). Les lettres a, b ou c associées aux chiffres indiquent les différences significatives entre traitements à 5% de confiance. Les valeurs en pourcentage indiquent l'écart d'amélioration par rapport au témoin.

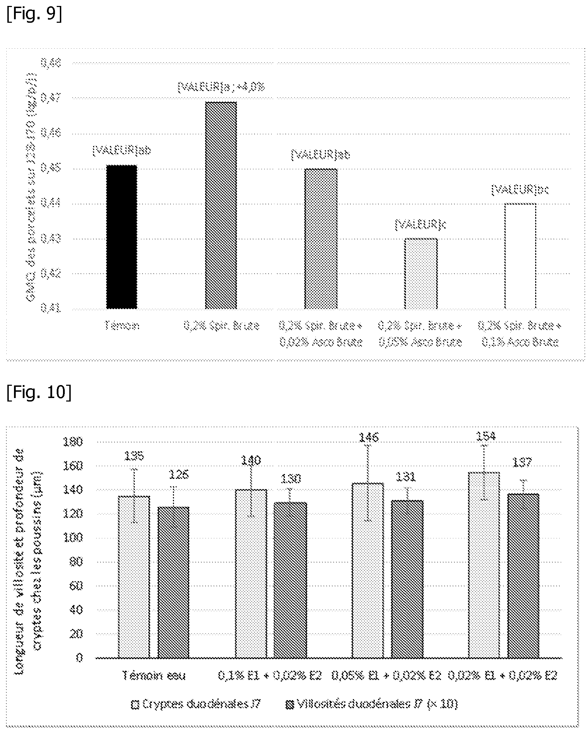

La figure 9 représente le gain moyen quotidien (GMQ) sur la phase de 1er âge entre J28 et J710 en kg/porcelet/j des animaux ayant reçu l'aliment témoin (Témoin T1), la *Spirulina platensis* brute à 0,2% (0,2% Spir. Brute T2), l'association *Spirulina platensis* brute et *d'Ascophyllum nodosum* brute à respectivement 0,2% et 0,02% (0,2% Spir. Brute + 0,02 % Asco Brute T3), à respectivement 0,2% et 0,05% (0,2% Spir. Brute + 0,05 % Asco Brute T4) ou à respectivement 0,2% et 0,1% (0,2% Spir. Brute + 0,1 % Asco Brute T5) dans les conditions de l'exemple 3 (ne faisant pas partie de l'invention). Les lettres a, b ou c associées aux chiffres indiquent les différences significatives entre traitements à 5% de confiance. Les valeurs en pourcentage indiquent l'écart d'amélioration par rapport au témoin.

La figure 10 représente la longueur des villosités duodénales et profondeur des cryptes duodénales en micromètre de poussins de 7 jours ayant reçu de l'eau de boisson normale (témoin : eau) ou de l'eau mélangée avec de l'hydrolysat protéique de *Spirulina platensis* (E1) et de l'extrait polysaccharidique *d'Ascophyllum nodosum* (E2) à respectivement 0,1% de E1+ 0,02% de E2, 0,05% de E1 + 0,02% de E2 ou 0,02% de E1 + 0,02% de E2 dans les conditions de l'exemple 4.

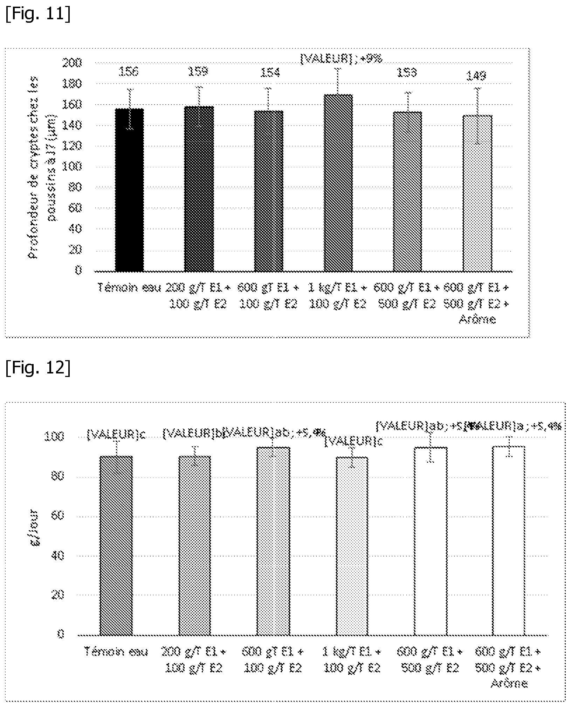

La figure 11 représente la profondeur des cryptes duodénales en micromètre mesurées chez les poussins de 7 jours ayant reçu une alimentation normale (témoin : rafle + eau - T1) ou un aliment pourvu de rafles mélangées avec de l'hydrolysat protéique de *Spirulina platensis* (E1) et de l'extrait polysaccharidique *d'Ascophyllum nodosum* (E2) à respectivement 200 g / T d'aliment de E1+ 100 g / T d'aliment de E2 (T2), 600 g/T de E1 + 100 g/T de E2 (T3), 1000 g/T de E1 + 100 g/T E2 (T4), 600 g/T de E1 + 500 g/T de E2 (T5) ou 600 g/T E1 + 500 g/T de E2 + Arôme (T6) dans les conditions de l'exemple 4. Les valeurs en pourcentage indiquent l'écart d'amélioration par rapport au témoin.

La figure 12 représente le gain moyen quotidien (GMQ) sur la phase entre J27 et J35 en g/j chez des poulets de chair ayant reçu pendant 35 jours une alimentation normale (témoin : rafle + eau - T1) ou un aliment pourvu de rafles mélangées avec de l'hydrolysat protéique de *Spirulina platensis* (E1) et de l'extrait polysaccharidique *d'Ascophyllum nodosum* (E2) à respectivement 200 g / T d'aliment de E1+ 100 g / T d'aliment de E2 (T2), 600 g/T de E1 + 100 g/T de E2 (T3), 1000 g/T de E1 + 100 g/T E2 (T4), 600 g/T de E1 + 500 g/T de E2 (T5) ou 600 g/T E1 + 500 g/T de E2 + Arôme (T6) dans les conditions de l'exemple 4. Les lettres a, b ou c associées aux chiffres indiquent les différences significatives entre traitements à 5% de confiance. Les valeurs en pourcentage indiquent l'écart d'amélioration par rapport au témoin.

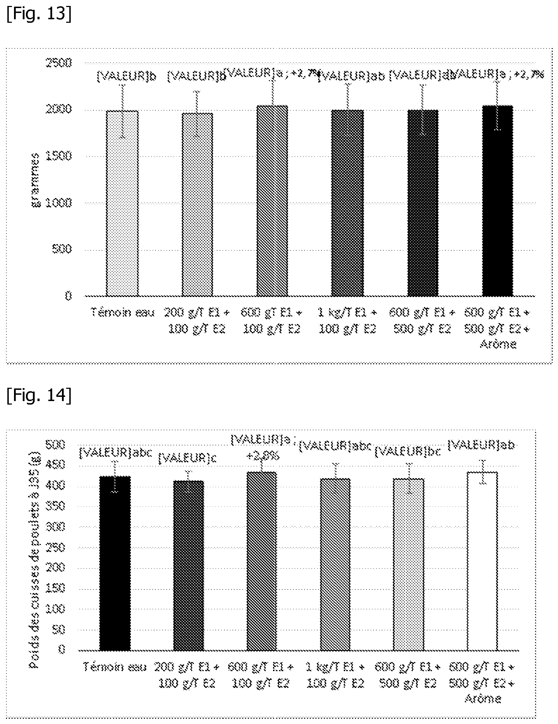

La figure 13 représente le poids vif final à J35 en gramme des poulets de chair ayant reçu l'alimentation normale (témoin : rafle + eau - T1) ou un aliment pourvu de rafles mélangées avec de l'hydrolysat protéique de *Spirulina platensis* (E1) et de l'extrait polysaccharidique *d'Ascophyllum nodosum* (E2) à respectivement 200 g / T d'aliment de E1+ 100 g / T d'aliment de E2 (T2), 600 g/T de E1 + 100 g/T de E2 (T3), 1000 g/T de E1 + 100 g/T E2 (T4), 600 g/T de E1 + 500 g/T de E2 (T5) ou 600 g/T E1 + 500 g/T de E2 + Arôme (T6) dans les conditions de l'exemple 4. Les lettres a, b ou c associées aux chiffres indiquent les différences significatives entre traitements à 5% de confiance. Les valeurs en pourcentage indiquent l'écart d'amélioration par rapport au témoin.

La figure 14 représente le poids des cuisses à J35 en gramme des poulets de chair ayant reçu l'alimentation normale (témoin : rafle + eau - T1) ou un aliment pourvu de rafles mélangées avec de l'hydrolysat protéique de *Spirulina platensis* (E1) et de l'extrait polysaccharidique *d'Ascophyllum nodosum* (E2) à respectivement 200 g / T d'aliment de E1+ 100 g / T d'aliment de E2 (T2), 600 g/T de E1 + 100 g/T de E2 (T3), 1000 g/T de E1 + 100 g/T E2 (T4), 600 g/T de E1 + 500 g/T de E2 (T5) ou 600 g/T E1 + 500 g/T de E2 + Arôme (T6) dans les conditions de l'exemple 4. Les lettres a, b ou c associées aux chiffres indiquent les différences significatives entre traitements à 5% de confiance. Les valeurs en pourcentage indiquent l'écart d'amélioration par rapport au témoin.

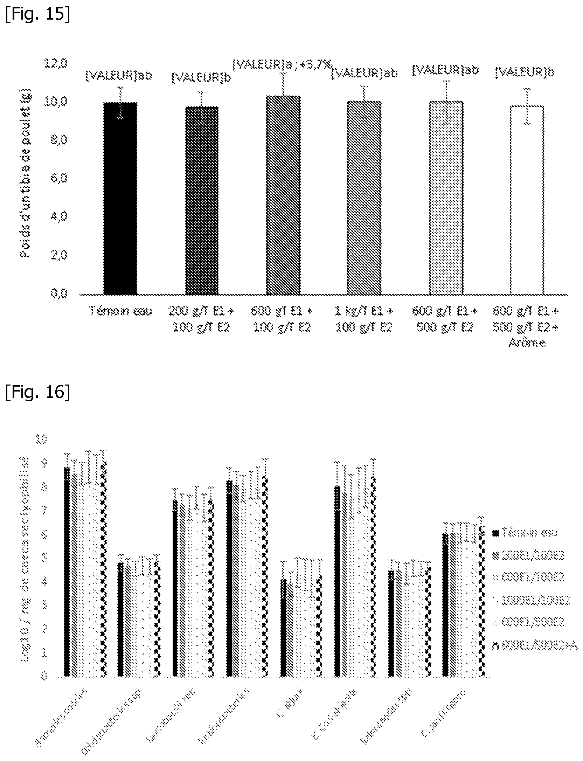

La figure 15 représente le poids d'un tibia à J35 en gramme des poulets de chair ayant reçu l'alimentation normale (témoin : rafle + eau - T1) ou un aliment pourvu de rafles mélangées avec de l'hydrolysat protéique de *Spirulina platensis* (E1) et de l'extrait polysaccharidique *d'Ascophyllum nodosum* (E2) à respectivement 200 g / T d'aliment de E1+ 100 g / T d'aliment de E2 (T2), 600 g/T de E1 + 100 g/T de E2 (T3), 1000 g/T de E1 + 100 g/T E2 (T4), 600 g/T de E1 + 500 g/T de E2 (T5) ou 600 g/T E1 + 500 g/T de E2 + Arôme (T6) dans les conditions de l'exemple 4.

La figure 16 représente le nombre de bactéries totales, de bifidobactéries spp, de lactobacilli spp, d'entérobactéries, de Clostridium jejuni, de *Escherichia. Coli-shigella,* de salmonelles spp et de *Clostridium perfringens* à J35 dans le caecum des poulets en log/mg de caecum sec lyophilisé chez des poulets de chair ayant reçu l'alimentation normale (témoin : rafle + eau - T1) ou un aliment pourvu de rafles mélangées avec de l'hydrolysat protéique de *Spirulina platensis* (E1) et de l'extrait polysaccharidique d'*Ascophyllum nodosum* (E2) à respectivement 200 g / T d'aliment de E1+ 100 g / T d'aliment de E2 (T2), 600 g/T de E1 + 100 g/T de E2 (T3), 1000 g/T de E1 + 100 g/T E2 (T4), 600 g/T de E1 + 500 g/T de E2 (T5) ou 600 g/T E1 + 500 g/T de E2 + Arôme (T6) dans les conditions de l'exemple 4.

**Exemple 1 : préparation des extraits**

Exemple 1a : extrait de spiruline (E1)

100g de spiruline brute (*Spirulina platensis)* déshydratée sous forme de poudre ayant une teneur en protéines supérieure ou égale à 60% et une teneur en Phycocyanine supérieure ou égale à 8% est extraite par macération dans 900g d'eau à une température de 60°C pendant une durée comprise entre 8 heures et 6 heures, plus avantageusement entre 2 heures et 4 heures. L'extrait protéique obtenu (liquide) en solution aqueuse est hydrolysé à l'aide de l'enzyme endoprotéase à sérine subtilisine commercialisée sous la dénomination commerciale Novo-Pro® D à une température de 60°C et à un pH de 8 de façon à obtenir un hydrolysat protéique de spiruline.

La composition de la spiruline brute pour 100g est rassemblée dans le tableau 1 suivant :

Tableau 1

| Constituants | Teneur en g |
|---|---|
| Eau | 7,10 |
| Matière sèche | 92,90 |

(suite)

| Constituants | Teneur en g |
|---|---|
| Matière minérale | 6,64 |
| Matière organique | 86,26 |
| Protéines brutes | 67,50 |

La composition de l'extrait protéique de spiruline pour 100g de spiruline brute est rassemblée dans le tableau 2 suivant :

Tableau 2

| Constituants | Teneur en g/l |
|---|---|
| Matière sèche | 61,0 |
| Matière minérale | 8,7 |
| Matière organique | 52,3 |
| Protéines | 45,2 |
| dont Phycocyanine | 10,9 |

La composition en polypeptides et peptides solubilisés de l'hydrolysat protéique de spiruline à partir de 100g de spiruline brute est rassemblée dans le tableau 3 suivant :

Tableau 3

| Constituants en fonction du poids moyen (PM) en Da | Teneur en g/l |
|---|---|
| PM> 20 000 Da | 1,4 |
| PM de 10 000 à 20 000 Da | 0,6 |
| PM de 5 000 à 10 000 Da | 0,4 |
| PM de 1 000 à 5 000 Da | 4,4 |
| PM de 500 à 1 000 Da | 6,8 |
| PM de 150 à 500 Da | 14,1 |
| PM < 150 Da | 17,5 |

La composition en acides aminés (PM < 150 Da) solubilisés de l'hydrolysat protéique de spiruline à partir de 100g de spiruline brute est rassemblée dans le tableau 4 suivant :

Tableau 4

| Constituants en fonction du poids moyen (PM) en Da | Teneur en g/l |
|---|---|
| Acide aspartique | 0,3 |
| Thréonine | 0,8 |
| Sérine | 0,9 |
| Acide glutamique | 0,7 |
| Proline | <0,2 |
| Glycine | 0,3 |
| Alanine | 1,4 |
| Cystine | <0,2 |
| Valine | 1,2 |

(suite)

| Constituants en fonction du poids moyen (PM) en Da | Teneur en g/l |
|---|---|
| Méthionine | 0,6 |
| Isoleucine | 0,8 |
| Leucine | 2,4 |
| Tyrosine | 1,2 |
| Phénylalanine | 1,4 |
| Histidine | 0,3 |
| Lysine | 1,0 |
| Arginine | 1,6 |

Exemple 1b : extrait d'Ascophyllum nodosum (E2)

200g *d'Ascophyllum nodosum* brut déshydraté sous forme de poudre est extrait par macération dans 800g d'eau à une température comprise entre 45°C et 90°C pendant une durée comprise entre 2 heures et 4 heures. L'extrait saccharidique obtenu (liquide) est clarifié par décantation ou essorage.

La composition de *l'Ascophyllum nodosum* brute pour 100g est rassemblée dans le tableau 5 suivant :

Tableau 5

| Constituants | Teneur en g |
|---|---|
| Eau | 13,5 |
| Matière sèche | 86,5 |
| Matière minérale | 26,4 |
| Matière organique | 60,1 |
| Glucides | 54,5 |
| Protéines | 5,6 |
| Cadmium | < 1 ppm |
| Plomb | < 2 ppm |
| Mercure | < 0,02 ppm |
| Fluor | < 75 ppm |
| Arsenic | 33,5 ppm |

La composition de l'extrait saccharidique liquide clarifié *d'Ascophyllum nodosum* pour 100g *d'Ascophyllum nodosum* brute est rassemblée dans le tableau 6 suivant :

Tableau 6

| Constituants | Teneur en g/l |
|---|---|
| Matière sèche | 143 |
| Matière minérale | 50 |
| Matière organique | 93 |
| Glucides | 86,7 |
| Protéines | 6,3 |
| Cadmium | < 1 ppm |
| Plomb | < 2 ppm |

(suite)

| Constituants | Teneur en g/l |
|---|---|
| Mercure | < 0,02 ppm |
| Fluor | < 75 ppm |
| Arsenic | 5,2 ppm |

**Exemple 2 : Etude de l'effet de la composition selon l'invention sur les fermentations du rumen et la dégradation de la ration**

Les compositions selon l'invention comprenant :

- Une teneur en extrait de spiruline obtenu selon l'exemple 1a (E1) correspondant à 50g/j et une teneur en extrait *d'Ascophyllum nodosum* obtenu selon l'exemple 1b (E2) correspondant à 20g/j (50+20g /J E1 + E2);
- Une teneur en extrait de spiruline obtenu selon l'exemple 1a (E1) correspondant à 5g/j et une teneur en extrait *d'Ascophyllum nodosum* obtenu selon l'exemple 1b (E2) correspondant à 10g/j (5+10g :JE1 + E2);
- Une teneur en extrait de spiruline obtenu selon l'exemple 1a (E1) correspondant à 5g/j, une teneur en extrait *d'Ascophyllum nodosum* obtenu selon l'exemple 1b (E2) correspondant à 10g/j et une teneur en lithothamne (CalseaPowder Advance CPA) correspondant à 80g/j en mélange simple (CPA + E1 + E2) ;
- Une teneur en extrait de spiruline obtenu selon l'exemple 1a (E1) correspondant à 5g/j, une teneur en extrait *d'Ascophyllum nodosum* obtenu selon l'exemple 1b (E2) correspondant à 10g/j et une teneur en lithothamne (CalseaPowder Advance CPA) correspondant à 80g/j avec imprégnation des extraits sur le CPA (CPA + E1 + E2) ;

ont été testées.

Les compositions comparatives comprenant :

- Une teneur en extrait de spiruline obtenu selon l'exemple 1a (E1) correspondant à 50g/j ou à 5g/j (50g /J E1 ou 5g /J E1) ;
- Une teneur en extrait *d'Ascophyllum nodosum* obtenu selon l'exemple 1b (E2) correspondant à 20g/j ou à 10g/j (20g /J E2 ou 10g /J E2);
- Une teneur en lithothamne (CalseaPowder Advance CPA) correspondant à 80g/j (CPA) ;
- Une teneur en extrait de spiruline obtenu selon l'exemple 1a (E1) correspondant à 5g/j et une teneur en lithothamne (CalseaPowder Advance CPA) correspondant à 80g/j en mélange simple (CPA + E1) ;
- Une teneur en extrait *d'Ascophyllum nodosum* obtenu selon l'exemple 1b (E2) correspondant à 10g/j et une teneur en lithothamne (CalseaPowder Advance CPA) correspondant à 80g/j en mélange simple (CPA + E2) ;
- Une teneur en extrait de spiruline obtenu selon l'exemple 1a (E1) correspondant à 5g/j et une teneur en lithothamne (CalseaPowder Advance CPA) correspondant à 80g/j avec imprégnation de l'extrait sur le CPA (CPA + E1) ;
- Une teneur en extrait *d'Ascophyllum nodosum* obtenu selon l'exemple 1b (E2) correspondant à 10g/j et une teneur en lithothamne (CalseaPowder Advance CPA) correspondant à 80g/j avec imprégnation de l'extrait sur le CPA (CPA + E2) ;

ont été testées.

MATERIEL ET METHODE

L'approche est basée sur la technique de fermentations *in vitro* développée par le laboratoire de Nutrition Animale du Centre Mondial de l'Innovation Roullier. Le principe repose sur l'incubation de jus de rumen frais de vache laitière en mélange avec une salive artificielle préparée au niveau du laboratoire. Les compositions à tester et la ration (type hiver - fibreuse) sont incorporées dans des conditions similaires à la réalité (anaérobiose à 39°C) et sous agitation continue (afin de mimer les mouvements péristaltiques). La flore bactérienne en présence du substrat adapté reproduit les fermentations ruminales ce qui permet de suivre la production de gaz ainsi que les produits terminaux tels que les AGV, le N-NH$_3$ en fonction des objectifs de l'étude.

Pour évaluer l'effet de la composition selon l'invention sur les fermentations du rumen et la dégradation de la ration, du jus de rumen est prélevé sur 3 vaches fistulées nourries avec une ration à base d'ensilage de maïs, de foin et de concentrés (mélange de céréales). Pour reproduire les conditions physiologiques, une ration à base d'ensilage de maïs, de foin et de concentré énergétique (50 :30 :20) est mise à incuber dans l'inoculum composé de jus de rumen et de salive artificielle. La proportion jus de rumen/salive artificielle est de 1:2. Chaque essai se déroule sur 24h avec un suivi intermédiaire à 6h de fermentation. Enfin, le pH initial est ajusté à 6,5 pour garantir les conditions physiologiques du rumen ou à 6,2 pour simuler les conditions favorables à l'acidose, avec de l'acide lactique à 80%. Chaque flacon de 100 ml à incuber était composé de :

- 20 ml de jus de rumen filtré
- 40 ml de salive artificielle (pouvoir tampon : Substances tampons : carbonate de Na et d'ammonium ; Macro-éléments : Na, P, K et Mg ; Micro-éléments : Ca, Mn, Co et Fe)
- 0,5 g de substrat broyé à 1mm : le substrat peut être une ration hiver (50% ensilage de maïs + 30% de foin + 20% concentré énergétique (constitué de 20% blé, 20% maïs, 20% orge, 20% pulpe de betterave, 15% son de blé et 5% mélasse) ou une ration acidogène (33% ensilage de maïs + 17% de tourteaux de soja + 50% concentré énergétique (constitué de 20% blé, 20% maïs, 20% orge, 20% pulpe de betterave, 15% son de blé et 5% mélasse) et
- La composition à tester

Après 6 et 24h d'incubation à 39,5°C, les flacons sont placés dans la glace pour stopper les réactions. Le pH est mesuré directement à la sortie de l'incubateur avant filtration.

Le substrat est séparé du jus de rumen par centrifugation (20 min à 4 000 tours par min) pour récupérer 50 ml de jus destiné au dosage des acides gras volatiles (AGV) dans le milieu. Le substrat récupéré est lyophilisé pendant 48h puis broyé pour les dosages des fibres (Fibres insolubles dans les détergents neutres (NDF) et Fibres insolubles dans les détergents acides (ADF)).

L'enregistrement continu de la pression dans les flacons permet de déterminer le volume de gaz produit en ml/g de matière sèche (MS). Enfin, sur la purge de chaque module d'enregistrement de la production de gaz, un sac collecteur est adapté afin de récupérer un échantillon pour permettre l'analyse de la composition des gaz produits après 24h d'incubation.

RESULTATS

- Suivi de la production de gaz :

La fermentation du substrat par la flore du rumen entraîne la production de gaz principalement le $CO_2$ et le méthane ($CH_4$). Les figures 1, 3 et 4 présentent la concentration et le volume de $CH_4$ produit après 24h de fermentation.

Les extraits E1 et E2 montrent une action synergique sur la production de $CH_4$ avec des réductions de 40% et de 36%, respectivement pour la concentration et le volume, pour les apports les plus hauts. L'impact est proportionnel à la dose d'application avec une baisse de 16% du volume aux doses faibles (figure 1). Ces traitements inhibent l'activité fermentaire d'une partie de la flore du rumen.

L'association des extraits en simple mélange avec le CPA (lithothamne) est le traitement le plus efficace pour limiter la production de $CH_4$ (-31% en volume) par rapport aux extraits seuls ou au CPA seul ou en mélange avec un des extraits (figure 3). Les modalités ont un effet marqué sur ces paramètres ($p < 0,001$).

Globalement, l'association des extraits imprégnés sur du CPA réduit de 19% le volume de $CH_4$ produit lors des fermentations du rumen (figure 4).

Ainsi, au cours de la fermentation, la production de $CH_4$ est réduite par l'ajout des compositions selon l'invention. Cette diminution confirme l'inhibition directe des bactéries méthanogènes et des protozoaires. En effet, l'association de ces 2 familles est responsable de 9 à 25% de la production du $CH_4$. L'ajout de la composition selon l'invention devrait permettre de limiter les pertes d'énergie puisque la production de $CH_4$ entraîne environ 10% de perte de l'énergie consommée par l'animal.

-Paramètres de la ration

La figure 2 présente les digestibilités de la matière sèche (dMS), de l'ADF (dADF) et du NDF (dNDF) après 24h de fermentation. L'action synergique des extraits permet d'améliorer entre 20% et près de 40% la dégradation de la ration selon la dose d'application et le paramètre considéré.

La composition selon l'invention permet donc d'améliorer la dMS. La ration est donc mieux consommée par la flore du rumen qu'elle soit cellulolytique ou amylolytique. La composition selon l'invention augmente également la dADF et la dNDF. Elle favorise la dégradation et l'utilisation des fibres de la ration dans ce cas riche en fourrage (80%) avec de l'ensilage de maïs et du foin.

CONCLUSION

L'effet de la composition selon l'invention sur les fermentations du rumen et la dégradation de la ration en conditions physiologiques a donc pu être évalué. Cette composition permet donc un fonctionnement optimal du rumen via l'optimisation des fermentations ruminales. Elle réduit la production de $CH_4$ entérique Elle inhibe donc probablement une partie de la flore, les protozoaires qui sont associés aux bactéries méthanogènes. Cette défaunation permet de limiter les émissions de $CH_4$ réduisant ainsi les pertes d'énergie pour l'animal, ce qui est essentiel pour les performances des vaches laitières.

La composition selon l'invention améliore significativement la dégradation de la ration. En effet, les digestibilités de la matière sèche (MS) et des fibres (NDF et ADF) augmentent par rapport au témoin sans supplémentation. Ce produit permet une meilleure utilisation et dégradation de la fibre par la flore du rumen dont l'effet est avéré et reproductible.

En revanche l'utilisation d'E1 et d'E2 seuls montre un effet moins important sur la production de $CH_4$ ce qui démontre bien que l'utilisation de ces seuls composés n'est pas suffisante pour améliorer de façon significative le métabolisme microbien et donc l'utilisation des rations.

**Exemple 3** (ne faisant pas partie de l'invention) : **Etude de l'effet de la composition selon l'invention sur les porcelets**

Les compositions selon l'invention comprenant :

- 0,2% en poids de spiruline *(Spirulina platensis)* brute et 0,02% en poids *d'Ascophyllum nodosum* brute (T3) ;
- 0,2% en poids de spiruline *(Spirulina platensis)* brute et 0,05% en poids *d'Ascophyllum nodosum* brute (T4) ;
- 0,2% en poids de spiruline *(Spirulina platensis)* brute et 0,1% en poids *d'Ascophyllum nodosum* brute (T5) ;

ont été testées.

Les compositions comparatives comprenant :

- 0,2% en poids de spiruline *(Spirulina platensis)* brute (T2) ;
- aucune algue (témoin T1) ;

ont été testées.

MATERIEL ET METHODE

Elevage de 28 à 70 jours d'âge, porcelets mâles uniquement. Deux bâtiments : bâtiment 1 : $5T \times 3C \times 18P$ - bâtiment 2 : $5T \times 3C \times 18P$. 51 (bât 1) + 51 (bât 2) = 108 porcelets par traitement, soit 540 porcelets au total (T = Traitement, C = Cages et P = Porcelets). Distribution des produits sur toute la période d'essai. L'alimentation de ces porcelets consiste en un aliment 1er âge puis un aliment 2eme âge.

Le sevrage du porcelet se caractérise par une réduction de l'ingéré, une période d'anorexie durant 2 à 4 jours induisant, s'il elle est mal géré, des lésions intestinales pouvant produire une inflammation sévère et des lésions tissulaires.

Contrôler de manière précoce l'inflammation intestinale est un défi majeur dans la gestion des troubles intestinaux des porcelets durant cette période difficile de leur vie.

Les changements morphométriques et fonctionnels peuvent également être causés par des facteurs nutritionnels présents dans certains régimes ou autres produits associés à la formulation.

A titre d'exemple, l'utilisation du tourteau de soja dans l'aliment du porcelet peut provoquer des fermentations dans l'intestin ainsi que des diarrhées. En effet, le tourteau soja contient des antigènes qui induisent une inflammation sévère et des lésions tissulaires. C'est pour cette raison que son inclusion est souvent limitée dans les aliments destinés aux porcelets. Ces porcelets ont souvent des villosités intestinales endommagées entrainant une mauvaise absorption des nutriments.

L'objectif de cet exemple est d'évaluer l'effet d'une supplémentation en composition selon l'invention sur le système immunitaire (protéines dans le sang) et sur la morphologie des villosités intestinales.

RESULTATS

Immunité sanguine : Les figures 5 et 6 représentent respectivement les teneurs de Béta-1-globuline (protéines sériques) et en protéines totales dans le sang des porcelets à J 70. La β1-globuline est un polypeptide qui existe sous une forme libre et une forme liée aux membranes des cellules. Ce polypeptide joue un rôle important dans les défenses immunitaires.

On remarque que la quantité de protéines sériques du sang augmente (p.val = 0,09) avec la spiruline seule ou en mélange avec 0,02% et 0,05% d'*Ascophyllum.* A cet effet, on observe une augmentation importante jusqu'à 19,7% pour la composition contenant 0,2% Spiruline brute + 0,02% *Ascophyllum* brute ou bien 12,8% pour la composition contenant 0,2% Spiruline brute + 0,05% *Ascophyllum* brute. La composition contenant 0,2% Spiruline brute + 0,02% *Ascophyllum* brute semble donc être la plus efficace.

De même, la quantité de protéines totales du sang augmente avec la spiruline seule ou bien avec la composition contenant 0,2% Spiruline brute + 0,02% *Ascophyllum* brute. Un effet intéressant est particulièrement observé pour la composition T3 (0,2% Spiruline brute + 0,02% Ascophyllum brute) avec une augmentation de 4,3%.

Longueur des villosités intestinales à J70 : De nombreuses études morphologiques de la muqueuse gastro-intestinale ont été entreprises pour comprendre les changements du fonctionnement intestinal pendant le développement des porcelets et particulièrement autour du sevrage. Le sevrage induit des changements rapides, transitoires et importants de la physiologie du porcelet mais également une maturation des capacités intestinales.

Dans notre étude, des mesures morphométriques ont été effectuées à J70 sur des échantillons d'intestin grêle et les résultats sont présentés dans la figure 7.

La supplémentation avec la composition selon l'invention a augmenté la longueur des villosités jéjunales sans effet majeur sur les villosités duodénales.

Les traitements contenant les plus fortes doses d'Ascophyllum augmentent (p.val = 0,08) la longueur des villosités du jéjunum jusqu'à 15,4% avec la composition contenant 0,2% Spiruline brute + 0,05% Ascophyllum brute (composition la plus intéressante).

Une supplémentation en composition selon l'invention peut donc moduler la réponse inflammatoire *via* l'augmentation des villosités jéjunales *via* l'apparition des sucres simples probablement ou des peptides (issues des réactions enzymatiques dans des sites d'absorption spécifiques) qui évitent ainsi les dommages associés au stress nutritionnel post sevrage.

Conclusions sur la santé et conclusion générale :

Des effets positifs avec la composition selon l'invention ont été observés notamment sur la longueur des villosités du jéjunum des porcelets.

Une modulation de la réponse immunitaire sur l'intégrité de la muqueuse intestinale a été ainsi été observée dans nos expériences avec un effet de la supplémentation en composition selon l'invention dans un élevage de porcelets mâles uniquement de 28 à 70 jours d'âge (post - sevrage).

Les résultats obtenus sont intéressants car la consommation journalière est très différente entre les différentes compositions entre J28 et J42 (période de 1er âge) avec une p. Value très significative (0,004) comme le montre la figure 8.

En effet, la consommation de l'aliment a été réduite soit en raison de la période de transition (post-sevrage) dans nos lots d'essais, soit probablement en raison du goût ou de l'odeur de la composition selon l'invention ; Toutefois, malgré cette réduction de l'ingéré, les résultats restent positifs sur la santé d'autant plus pour la composition T3 (0,2% Spiruline brute + 0,02% Ascophyllum brute) dont le gain moyen quotidien (GMQ) est équivalent au témoin (cf. figure 9) tout en étant corrélé à une consommation journalière très faible (-11,8 %/ T1).

**Exemple** 4 : **Etude de l'effet de la composition selon l'invention sur les poulets de chair**

Les compositions selon l'invention comprenant :

- 

    200 g d'extrait de Spiruline obtenu selon l'exemple 1a (E1) / T d'aliment + 100 g d'extrait d'Ascophyllum obtenu selon l'exemple 1b (E2) / T d'aliment (T2) ;

- 

    600 g/T de E1 + 100 g/T de E2 (T3) ;

- 

    1000 g/T de E1 + 100 g/T E2 (T4) ;

-

600 g/T E1 + 500 g/T de E2 (T5) ;

-

600 g/T E1 + 500 g/T de E2 + 0,05g/kg d'arôme commercialisé par Kaesler (T6) ;

ont été testées.

Les compositions comparatives comprenant :

- aucune algue et de l'eau (témoin T1) ;

ont été testées.

MATERIEL ET METHODE

L'aliment a été formulé à 95% des besoins nutritionnels des poulets mâles de souche Ross 308 d'Aviagen. Il a été distribué *adlibitum* pendant 35 jours. L'essai était composé de 6 traitements alimentaires comprenant des rafles et une des compositions indiquée ci-dessus (T1 à T6). Ces traitements ont été distribués pendant toute la période d'élevage.

Un ensemble de paramètres imputables à l'intestin ont été relevés en cours (J7 et J15) et en fin d'essai à J35.

RESULTATS

Le paramètre relevé en phase de démarrage (J7) et de croissance (J15) est l'étude histologique de la muqueuse intestinale. La longueur des villosités et la profondeur des cryptes au niveau du duodénum, du jéjunum et de l'iléon ont été mesurées. Ainsi l'étude de la morphologie intestinale au démarrage a montré une tendance (p.val = 0,16) à une augmentation de la profondeur des cryptes duodénales à J7 (Figure 11). La profondeur des cryptes des poussins du traitement T4 (1000 g/T E1 + 100 g/T E2) présente une augmentation de 9% comparativement au traitement témoin T1. Des résultats similaires ont pu être observés sur la même phase d'élevage dans un essai précédent qui avait en effet démontré une tendance à une augmentation de la longueur des villosités (p.val = 0,11) et de la profondeur des cryptes duodénales (p.val = 0,15) à J7, de poussins ayant reçu la composition selon l'invention dans leur eau de boisson (Figure 12). A dose d'extrait d'Ascophyllum égale dans l'eau (0,02%), les cryptes et les villosités ont accru avec la diminution de la dose d'extrait de spiruline de 0,1 à 0,02%. Contrairement à l'apport dans l'aliment, il semblerait que la dose d'extrait de spiruline apportée dans l'eau de boisson n'ait pas besoin d'être maximale pour favoriser la croissance des villosités et la profondeur des cryptes. La dose dépendra donc de la forme d'apport choisie.

Ces variations dans la longueur des villosités et dans la profondeur des cryptes sont un bon indicateur de la digestion des nutriments et des capacités d'absorption de l'intestin grêle. En effet, de façon plus importante encore, le développement de la muqueuse intestinale se fait très rapidement après l'éclosion dans les 7 premiers jours de vie de l'animal. En particulier, ce développement s'effectue au début de l'intestin dans le duodénum et cela bien que les niveaux sont différents entre segments intestinaux. Ainsi, le volume des villosités dans le duodénum atteint un plateau après 7 jours alors qu'il continue à croître dans le jéjunum et l'iléon après 7 jours. Or, le poids vif à 42 jours d'âge est corrélé au poids vif à 21 jours, lui-même corrélé au poids vif à 7 jours, lui-même en relation avec la bonne capacité absorptive en début de vie. Des villosités larges, des microvillosités plus nombreuses et des cellules épithéliales plus actives au niveau du duodénum et du jéjunum au début de la vie des poulets suggèrent une grande surface absorptive et une fonction intestinale active, qui permettent une croissance plus rapide et meilleure des poulets après l'éclosion.

Les données de performances (poids et consommations alimentaires) ont été relevées en période de finition à J27 et J35. Grâce à cela, l'indice de consommation (IC) et le gain moyen quotidien (GMQ) des poulets ont pu être calculés. Les performances en finition mesurées entre J27 et J35 ont ainsi montré une amélioration du $GMQ_{27\text{-}35}$ sur cette période avec l'apport de la composition selon l'invention (Figure 12). Sur la dernière semaine d'élevage, l'apport du mix d'extraits, en particulier de la dose de 600 g/T de E1 cumulée à 100 ou 500 g/T de E2 a permis d'augmenter le $GMQ_{27\text{-}35}$ d'environ 5,4% par rapport au témoin eau (p.val = 0,03). De même, cet essai a démontré une augmentation du poids vif (PV) final à J35 (Figure 13). De la même façon que pour le $GMQ_{27\text{-}35}$, le PV à J35 est amélioré d'environ 2,7% avec une dose de 600 g/T d'extrait de spiruline (E1) cumulée à une dose de 100 ou 500 g/T d'extrait d'Ascophyllum (E2). Cette amélioration du PV final est donc due à une augmentation du GMQ sur la dernière semaine d'élevage. Cela semble indiquer que dans le cas d'une distribution d'extraits dans l'aliment, celle-ci devrait se faire sur l'ensemble de la période d'élevage pour obtenir ce résultat.

Afin de comprendre d'où provenait cette amélioration du PV final, des corrélations ont été menées entre ce PV et le poids des organes et pièces prélevés et pesés pendant cet essai. Une corrélation de 94% a été mesurée entre le PV et le poids des cuisses des poulets. Une corrélation moyenne de 50% a été mesurée entre le PV et les filets ou les tibias. Les pièces de viande, en particulier les cuisses et le squelette semblent donc expliquer la prise de poids.

La pesée des pièces de découpe les plus valorisées en alimentation humaine (filet et cuisses) ont montré une tendance (p.val = 0,17) à l'augmentation du poids des cuisses selon les traitements (Figure 14). La figure 14 montre donc que les poulets ayant reçu 600 g/T de E1 + 100 g/T E2 présentent un poids de cuisses plus élevé de presque 3% par rapport au témoin eau. Les poulets de ce traitement un peu plus lourds en fin d'élevage ont également un poids de cuisse plus élevé que les poulets des autres traitements.

Comme le montre la figure 15, le poids des tibias a également eu tendance à augmenter (p.val = 0,18) de presque 4% avec l'apport de 600 g/T de E1 + 100 g/T de E2. Une telle prédisposition est relativement importante chez le poulet de chair dont la génétique sélectionnée avec précision au cours de ces dernières décennies a eu pour conséquence une amélioration des capacités de croissance des animaux. Le taux de croissance des poulets a en effet augmenté de 300% en cinquante ans, passant de 25 g / jour à 100 g / jour. Ce progrès a cependant eu pour conséquence la fragilisation de leurs pattes, trop faibles pour soutenir un tel poids aussi rapidement. Au bout de quarante jours d'élevage, cela peut représenter près de 30% des poulets présentant des problèmes de locomotions ou une impossibilité à marcher. La croissance de ces animaux est alors ralentie, les poulets n'ayant plus accès à la mangeoire. Indirectement ces problèmes représentent une perte économique pour les éleveurs. Il est donc important que la structure osseuse des tibias soit de qualité puisque c'est une composante qui va jouer sur le salaire des éleveurs.

Les paramètres relevés en phase de finition (J35) ou à l'abattage sont :

- à l'abattage, des échantillons de contenu caecal ont également été relevés afin de comptabiliser par qPCR les populations bactériennes suivantes :

  Populations bénéfiques :

  - Lactobacilles
  - Bifidobactéries

  Populations pathogènes :

  - *Clostridium perfringens*
  - Salmonelles spp
  - *Escherichia. Coli-shigella*
  - *Clostridium jejuni*
  - Entérobactéries

  Bactéries totales

Les populations bactériennes du caecum ont été dénombrées. Les résultats ont montré des modifications dans ces populations avec l'apport de la composition selon l'invention (Figure 16).

La figure 16 montre ainsi que les poulets ayant reçus 600 g/T de E1 + 100 g/T de E2 (T3) présentent le plus faible développement de populations bactériennes. Ceci est vrai particulièrement pour les bifidobactéries, les lactobacilles, les entérobactéries, *E. coli-shigella* et les salmonelles.

De ce fait, le nombre total de bactéries dans le caecum est de 8,59 Log10 pour les poulets de ce traitement quand ce nombre est compris entre 8,60 et 9,05 Log10 pour les autres traitements. Le traitement avec arôme n'est ici pas recommandé car il favorise le développement de ces populations. Une analyse discriminante couplée à une comparaison des distances de cette analyse par la méthode de Fisher a révélé que les populations du témoin et du traitement T3 sont significativement différentes (p.val = 0,04). En outre, nous remarquerons que l'ensemble des effets observés jusque ici entre les traitements T2, T3 et T4, à dose constante d'extraits d'*Ascophyllum* mais à dose croissante d'extrait de spiruline suivent non pas une régression linéaire (amélioration des paramètres avec la dose d'extrait de spiruline) mais une régression quadratique à plateau. Ainsi, la dose d'extrait de spiruline n'a pas besoin d'être maximale pour que des effets s'observent. A l'inverse une dose trop forte d'extrait de spiruline pourrait décroitre les résultats.

Conclusion générale

L'essai présenté ici, agrémenté de résultats d'un essai précédent a démontré l'intérêt de l'apport soit d'une dose de 0,02% d'extrait de spiruline + 0,02% d'extrait d'*Ascophyllum* dans de l'eau de boisson de poulets au démarrage de J0 à J7, soit d'une dose de 1000 g d'extrait de spiruline + 100 g d'extrait de spiruline / T d'aliment de poulets au même âge. Un complément alimentaire au démarrage pourrait en effet être proposé en ce sens qu'il favorise l'augmentation de la longueur des villosités et la profondeur des cryptes duodénales. Cet effet est en outre un bon indicateur de la digestion des nutriments et des capacités d'absorption de l'intestin grêle, fonctions relativement importantes au démarrage car elles peuvent conditionner la prise de poids des animaux et la modulation de l'immunité tout au long de leur vie. La dose préconisée dépendra de la forme d'apport : dans l'eau de boisson ou dans l'aliment.

De la même façon, ce complément alimentaire pourrait continuer d'être distribué jusqu'à la fin de la période d'élevage à une dose de 600 g d'extrait de spiruline + 100 g d'extrait d'Ascophyllum / T d'aliment afin d'obtenir un PV final plus élevé qui s'explique par un poids des cuisses plus important. Cette dose permet également d'obtenir un poids de tibia plus élevé favorable à une meilleure locomotion et une diminution des populations bactériennes caecales totales dont les bactéries pathogènes *E. coli shigella,* entérobactéries et salmonelles.

**Exemple 5** : **Etude de l'effet de la composition selon l'invention sur les fermentations du rumen et la dégradation de la ration**

Les compositions selon l'invention comprenant :

- un extrait *d'Ascophyllum nodosum* obtenu selon l'exemple 1b (AN- E) ;
- un extrait de *Spirulina platensis* obtenu selon l'exemple 1a (SP-E) ;
- un mélange (SAT-E) de 32% en poids d'extrait de spiruline obtenu selon l'exemple 1a (E1) et de 68% en poids d'extrait d'*Ascophyllum nodosum* obtenu selon l'exemple 1b (E2);
- de la *Spirulina platensis* sous forme brute (SP-B) ;
- de *l'Ascophyllum nodosum* sous forme brute (AN-B) ;
- un mélange (SAT-B) de 32% en poids *Spirulina platensis sous* forme brute et de 68% en poids *d'Ascophyllum nodosum* sous forme brute.

Les doses testées pour chacun des mélanges sont de 1 à 30 g/j (basse). La méthode est la même que celle de l'exemple 1 (technique de fermentations *in vitro* développée par le laboratoire de Nutrition Animale du Centre Mondial de l'Innovation Roullier).

RESULTATS :

Ils sont rassemblés dans les tableaux 7 et 8 suivants :

Tableau 7 : effets sur la production de méthane

|  | dose | $[CH_4]$ % | Volume de $CH_4$ (ml/g de MS) | *diff en $[CH_4]$* | *diff en volume* |
|---|---|---|---|---|---|
| Témoin | Aucune | 4,4 | 6,5 |  |  |
| **AN-B** | Basse | 4,1 | 6,24 | *-5%* | *-4%* |
| **SAT-B** | **Basse** | **3,1** | **4,83** | ***-29%*** | ***-26%*** |
| **SP-B** | Basse | 3,9 | 5,69 | *-10%* | *-12%* |
| **AN-E** | Basse | 4,2 | 6,20 | *-4%* | *-5%* |
| **SAT-E** | **Basse** | **3,7** | **5,75** | ***-15%*** | ***-12%*** |
| **SP-E** | Basse | 4,2 | 5,92 | *-4%* | *-9%* |

On observe un effet significatif de la dose d'application basse (<30g/l): $p<0,001$ sur la concentration en $CH_4$ du gaz pour le mélange qui est supérieur à celui des algues seules, ce qui démontre bien une synergie.

Le mélange des algues brutes est même plus intéressant que le mélange des extrait d'algues (-29% sur la concentration et -26% sur le volume).

Tableau 8 : effets sur la digestibilité de la ration (dMS) :

|  | dose | dMS % | *diff vs témoin* |
|---|---|---|---|
| Témoin | Aucune | 49 |  |
| **AN-B** | Basse | 49 | *0%* |
| **SAT-B** | **Basse** | **54,2** | ***11%*** |

(suite)

|  | dose | dMS % | *diff vs témoin* |
|---|---|---|---|
| **SP-B** | Basse | 50,3 | *3%* |
| **AN-E** | Basse | 49,1 | *0%* |
| **SAT-E** | **Basse** | **53,2** | *8%* |
| **SP-E** | Basse | 48,5 | *-1%* |

La composition selon l'invention contenant le mélange d'algue selon l'invention, que ce soit sous dans leur forme brute ou d'extrait, montrent une légère amélioration de la dMS contrairement aux algues seules. L'effet synergique sera potentiellement plus marqué en *in vivo* avec une consommation quotidienne.

En outre, aucun effet négatif sur les fermentations n'a été constaté aux doses testées.

**Revendications**

1. Composition pour la nutrition ou la boisson d'un animal non humain d'élevage, avantageusement d'un ruminant et/ou d'un monogastrique tel qu'une volaille ou un porc et/ou d'un animal aquatique tel qu'un poisson ou un crustacé, comprenant l'association d'un hydrolysat protéique de spiruline et d'un extrait polysaccharidique d'*Ascophyllum nodosum.*

2. Composition selon la revendication 1, **caractérisée en ce que** l' extrait polysaccharidique *d'Ascophyllum nodosum* est obtenu par extraction dans un solvant aqueux.

3. Composition selon l'une quelconque des revendications 1 ou 2, **caractérisée en ce que** le ratio en poids hydrolysat protéique de spiruline / extrait polysaccharidique *d'Ascophyllum nodosum,* est compris dans la gamme 0,02-100, avantageusement 0,2 - 15, en particulier 0,5 - 20.

4. Composition selon l'une quelconque des revendications 1 à 3, **caractérisée en ce qu'**elle comprend entre 0,01 et 0,5 % en poids par rapport au poids total de la composition, avantageusement entre 0,02 et 0,2% en poids par rapport au poids total de la composition, d'hydrolysat protéique de spiruline.

5. Composition selon l'une quelconque des revendications 1 à 4, **caractérisée en ce qu'**elle comprend entre 0,05 et 0,5 % en poids par rapport au poids total de la composition, avantageusement entre 0,01 et 0,1% en poids par rapport au poids total de la composition, d'extrait polysaccharidique *d'Ascophyllum nodosum.*

6. Composition selon l'une quelconque des revendications 1 à 5, **caractérisée en ce qu'**elle comprend en outre du lithothamne.

7. Utilisation de la composition selon l'une quelconque des revendications 1 à 6, pour améliorer la performance zootechnique d'un ruminant et/ou d'un omnivore par exemple un porc.

8. Utilisation de la composition selon l'une quelconque des revendications 1 à 6, pour diminuer l'émission de gaz à effet de serre, en particulier de méthane chez un ruminant.

9. Composition selon l'une quelconque des revendications 1 à 6, pour son utilisation pour améliorer le système immunitaire chez des animaux non humains d'élevage avantageusement des ruminants et/ou des monogastriques tels que les oiseaux par exemple les volailles ou les omnivores par exemple les porcs et/ou les animaux aquatiques tels que les poissons ou les crustacés , en particulier chez les poulets, les poussins, les porcs ou les porcelets.

**Patentansprüche**

1. Zusammensetzung für die Ernährung oder das Getränk eines nichtmenschlichen Nutztiers, vorteilhafterweise eines Wiederkäuers und/oder eines Monogastriers, wie beispielsweise Geflügel oder Schwein und/oder eines Wassertiers, wie beispielsweise Fisch oder Krustentier, umfassend die Assoziation eines Proteinhydrolysats von Spirulina und eines Polysaccharidextrakts von *Ascophyllum nodosum.*

2. Zusammensetzung nach Anspruch 1, **dadurch gekennzeichnet, dass** der Polysaccharidextrakt von *Ascophyllum nodosum* durch Extraktion in einem wässrigen Lösungsmittel erlangt wird.

3. Zusammensetzung nach einem der Ansprüche 1 oder 2, **dadurch gekennzeichnet, dass** das Gewichtsverhältnis von Spirulina-Proteinhydrolysat zu Polysaccharidextrakt von *Ascophyllum nodosum* in dem Bereich von 0,02-100, vorzugsweise 0,2-15, insbesondere 0,5-20, liegt.

4. Zusammensetzung nach einem der Ansprüche 1 bis 3, **dadurch gekennzeichnet, dass** sie zwischen 0,01 und 0,5 Gewichts-%, bezogen auf das Gesamtgewicht der Zusammensetzung, vorteilhafterweise zwischen 0,02 und 0,2 Gewichts-%, bezogen auf das Gesamtgewicht der Zusammensetzung, von Spirulina-Proteinhydrolysat umfasst.

5. Zusammensetzung nach einem der Ansprüche 1 bis 4, **dadurch gekennzeichnet, dass** sie zwischen 0,05 und 0,5 Gewichts-%, bezogen auf das Gesamtgewicht der Zusammensetzung, vorteilhafterweise zwischen 0,01 und 0,1 Gewichts-%, bezogen auf das Gesamtgewicht der Zusammensetzung, Polysaccharidextrakt von *Ascophyllum nodosum* enthält.

6. Zusammensetzung nach einem der Ansprüche 1 bis 5, **dadurch gekennzeichnet, dass** sie ferner Lithothamnium umfasst.

7. Verwendung der Zusammensetzung nach einem der Ansprüche 1 bis 6, zum Verbessern der zootechnischen Leistung eines Wiederkäuers und/oder eines Allesfressers, beispielsweise eines Schweins.

8. Verwendung der Zusammensetzung nach einem der Ansprüche 1 bis 6, um die Emission von Treibhausgasen, insbesondere von Methan, bei einem Wiederkäuer zu verringern.

9. Zusammensetzung nach einem der Ansprüche 1 bis 6, zur Verwendung zum Verbessern des Immunsystems bei nichtmenschlichen Nutztieren, vorzugsweise Wiederkäuern und/oder Monogastriern, beispielsweise Vögeln, zum Beispiel Geflügel, oder Allesfressern, beispielsweise Schweinen, und/oder Wassertieren, beispielsweise Fischen oder Schalentieren, insbesondere bei Hühnern, Küken, Schweinen oder Ferkeln.

## Claims

1. A composition for the nutrition or drink of a non-human farm animal, advantageously a ruminant animal and/or a monogastric animal such as a poultry or a pig and/or an aquatic animal such as a fish or a crustacean, comprising the combination of a protein hydrolyzate of spirulina and polysaccharide extract of *Ascophyllum nodosum.*

2. The composition according to claim 1, **characterized in that** the polysaccharide extract of *Ascophyllum nodosum* is obtained by extraction in an aqueous solvent.

3. The composition according to any one of claims 1 or 2, **characterized in that** the protein hydrolyzate of spirulina / polysaccharide extract of *Ascophyllum nodosum weight* ratio, is comprised within the range 0.02-100, advantageously 0.2 - 15, in particular 0.5 - 20.

4. The composition according to any one of claims 1 to 3, **characterized in that** it comprises between 0.01 and 0.5% by weight relative to the total weight of the composition, advantageously between 0.02 and 0.2% by weight relative to the total weight of the composition, of the protein hydrolyzate of spirulina.

5. The composition according to any one of claims 1 to 4, **characterized in that** it comprises between 0.05 and 0.5% by weight relative to the total weight of the composition, advantageously between 0.01 and 0.1% by weight relative to the total weight of the composition, of *Ascophyllum nodosum,* advantageously of *Ascophyllum nodosum* extract.

6. The composition according to any one of claims 1 to 5, **characterized in that** it further comprises lithothamnium.

7. A use of the composition according to any one of claims 1 to 6, for improving the zootechnical performance of a ruminant and/or an omnivore for example a pig.

8. The use of the composition according to any one of claims 1 to 6, in order to reduce the emission of greenhouse gases, in particular methane, in a ruminant animal.

9. The composition according to any one of claims 1 to 6, for its use for improving the immune system in non-human farm animals, advantageously ruminant animals and/or monogastric animals such as birds, for example poultry, or omnivore animals, for example pigs, and/or aquatic animals such as fish or crustaceans, in particular in chickens, chicks, pigs or piglets.

[Fig. 1]

[Fig. 2]

[Fig. 3]

[Fig. 4]

[Fig. 5]

[Fig. 6]

[Fig. 7]

[Fig. 8]

[Fig. 9]

[Fig. 10]

[Fig. 11]

[Fig. 12]

[Fig. 13]

[Fig. 14]

[Fig. 15]

[Fig. 16]

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2004080196 A
- WO 200122822 A
- EP 1570845 A
- KR 20200129841
- DE 19608563